# EUROPEAN PATENT APPLICATION

(11) **EP 4 534 681 A1**
(43) Date of publication of application: **09.04.2025**
(21) Application number: 23815902.4
(22) Date of filing: 24.05.2023
(51) Int. Cl.: C12N 15/86, A61K 35/76, A61K 38/46, A61P 35/00, C12N 15/09, C12N 15/12

(54) **VIRUS VECTOR AND CANCER CELL PROLIFERATION INHIBITOR COMPRISING SAME**

(30) Priority: 31.05.2022 JP 2022088235
(71) Applicant: Hikari Bio Inc., Takamatsu-shi, Kagawa, 761-8042 (JP)
(72) Inventor: YOSHIDA Tadashi, Takamatsu-shi, Kagawa 761-8042 (JP); HIROTA Norifumi, Takamatsu-shi, Kagawa 761-8042 (JP)
(74) Representative: Wilson Gunn
(86) International application number: PCT/JP2023/019342
(87) International publication number: WO 2023/234143

(57) **Abstract**

The invention relates to a viral vector formed or prepared such that a full-length cDNA having a stop codon of Kras is transduced into the first exon of Kras of a human cell genome DNA so that the viral vector can inhibit progression of cancer cells by directly editing a genome of cancer cells to repair a gene function.

## Description

### Technical Field

The present invention relates to a viral vector, and a cancer cell proliferation inhibitor comprising the same.

### Background Art

Cancers are a sort of diseases developed by accumulation of genetic mutations, and studies and developments of treating methods regarding molecular mechanisms of rendering cells abnormal have been conducted over long years to clarify molecular mechanisms for carcinogenesis to a considerable extent by virtue of various studies.

However, as some cancer cell properties vary with accumulation of various genetic mutations in association of cancer progression, any treatment effective in targeting on cancer cells alone has been not yet established. While, up to today, targeted therapy focusing on cell surface receptors or released growth factors has been developed, its effect is limited by a problem with accumulation of various genetic mutations, leading to a failure of establishment of any efficacious therapy.

Conventional genetic therapies comprising viral vector transduction rely on only random introduction of exogenous genes into genomes and, hence, adapt to loss-of-function mutation alone. So far, their practical application to the genetic treatment for cancers has been considered difficult thanks to efficiency drops due to random introduction into genomes and side effects on genes other than targeted ones.

Recently, there has been clarification of a genome editing tool called CRISPR-Cas9 that is the immunological mechanism of archaea ending up with the development of a genome editing tool for biological cells, making it possible to use a CRISPR-Cas9 system capable of repairing and reducing genetic functions and adeno-associated viral vector suitable for application to living organisms, resulting in the development of a new therapy that can provide a solution to problems associated with conventional gene treatments.

Referring here to recent overseas studies, there has been an animal experimentation one effective for treatment of Huntington's disease and myodystrophy using the CRISPR-Cas9 system, leading further to commencement of clinical studies using the CRISPR-Cas9 system. Japan's MHLW is also framing a plan enabling a genetic treatment using genome editing, which would be a promising treatment of cancers caused by accumulation of gene mutations.
Non-Patent Publication 1: Cia-Hin Lau, Yousin Suh, "In vivo genome editing in animals using AAV-CRISPR system: applications to translational research of human disease", F1000Research, 2017, Vol.6, p:2153.

### Summary of the Invention

### Prior-Art Problems

Up to now, treatments using genome editing technologies are still limited to hereditary diseases; any treatment by use of genome editing is still not established. The present invention has for its main purpose to make use of genome editing technologies such that, as an example, a genome is edited directly for colorectal cancer to repair a gene function thereby suppressing progression of cancer cells.

### Subject Matters

To provide a solution to such an object as mentioned above, the viral vector according to one aspect of the invention may be a viral vector formed or otherwise prepared such that a full-length cDNA with a stop codon of Kras is transduced into the first exon of Kras of a human cell genome DNA.

According to another aspect of the invention, the viral vector may be a viral vector formed or otherwise prepared such that a full-length cDNA with a stop codon of TP53 is transduced into the first exon of TP53 of a human cell genome DNA.

According to yet aspect of the invention, the viral vector may be a viral vector formed or otherwise prepared such that a full-length cDNA with a stop codon is transduced into the genome DNA at an exon site just before a transcription start point of a specific gene.

According to a further aspect of the invention, the viral vector may be a viral vector formed or otherwise prepared such that cDNA with a stop codon corresponding to the 2^{nd} to 4^{th} exon of Kras is transduced into the 2^{nd} exon of Kras of a genome DNA.

According to a further aspect of the invention, the viral vector may be a viral vector formed or otherwise prepared such that cDNA with a stop codon corresponding to the 2^{nd} to 4^{th} exon of Kras is transduced into the 2^{nd} exon of Kras of a genome DNA.

According to a further aspect of the invention, the viral vector may be a viral vector formed or otherwise prepared such that cDNA with a stop codon of up to the last site of a region translated from an MCR site of APC upon which gene mutations concentrate is transduced from around a site of commencement of the 15^{th} exon of APC of a genome DNA to the last of a gene protein coding region.

According to a further aspect of the invention, the present invention provides a cancer cell proliferation inhibitor characterized by comprising the viral vector as recited herein and a DNA nuclease.

### Advantages of the Invention

According to the viral vector and cancer cell proliferation inhibitor of the present invention, it is possible to edit a genome directly for a cancer cell and repair a gene function thereby inhibiting progress of cancer cells.

Unlikely conventional cancer treatments, the present invention has for an object to use a non-pathogenic viral vector while an influence upon normal cells is minimized to repair the function of a mutant gene responsible for making cancer cell abnormal whereby safer and everlasting gene therapy could be established.

### Brief Explanation of the Drawings

Fig. 1 is illustrative of the adeno-associated viral vector (AAV) disclosed herein.
Fig. 2 is schematically illustrative of the mechanism of the cancer cell proliferation inhibitor disclosed herein.
Fig. 3 is illustrative of the mechanism of repairing mutant genes.
Fig. 4 is illustrative of two adeno-associated viral vectors (AAVs) having Kras incorporated therein.
Fig. 5 is illustrative of two types of adeno-associated viral vectors (AAVs) having TP53 incorporated therein.
Fig. 6 is illustrative of two types of adeno-associated viral vectors (AAVs) having APC incorporated therein.
Fig. 7 is illustrative of a full-length sequence (Sequence No. 31) of Kras coupled to a special sequence (indicated by lower-case letters) designed for incorporation in the first exon of Kras in the genome of a cancer cell.
Fig. 8 is illustrative of a full-length sequence (Sequence No. 32) of Kras coupled to a special sequence (indicated by lower-case letters) designed for incorporation in the second exon of Kras in the genome of a cancer cell.
Fig. 9 is illustrative of a full-length sequence (Sequence No. 33) of TP53 coupled to a special sequence (indicated by lower-case letters) designed for incorporation in the first exon of TP53 in the genome of a cancer cell.
Fig. 10 is illustrative of a partial DNA sequence (Sequence No. 34) of TP53 coupled to a special sequence (indicated by lower-case letters) designed for incorporation in the fifth exon of TP53 in the genome of a cancer cell.
Fig. 11 is illustrative of a partial DNA sequency (Sequence No. 35) of TP coupled to a special sequence (indicated by lower-case letters) designed for incorporation in the 15^{th} exon of APC in the genome of a cancer cell.
Fig. 12 is illustrative of a location where Kras is inserted in Exon2 (Sequence No. 36), and a location where TP53 is inserted in Exon1 (Sequence No. 37).
Fig. 13 is illustrative of a location where Kras is inserted in Exon2 (Sequence No. 38), and a location where TP53 is inserted in Exon5 (Sequence No. 39).
Fig. 14 is illustrative of a location where APC is inserted in Exon15 (Sequence No. 40).

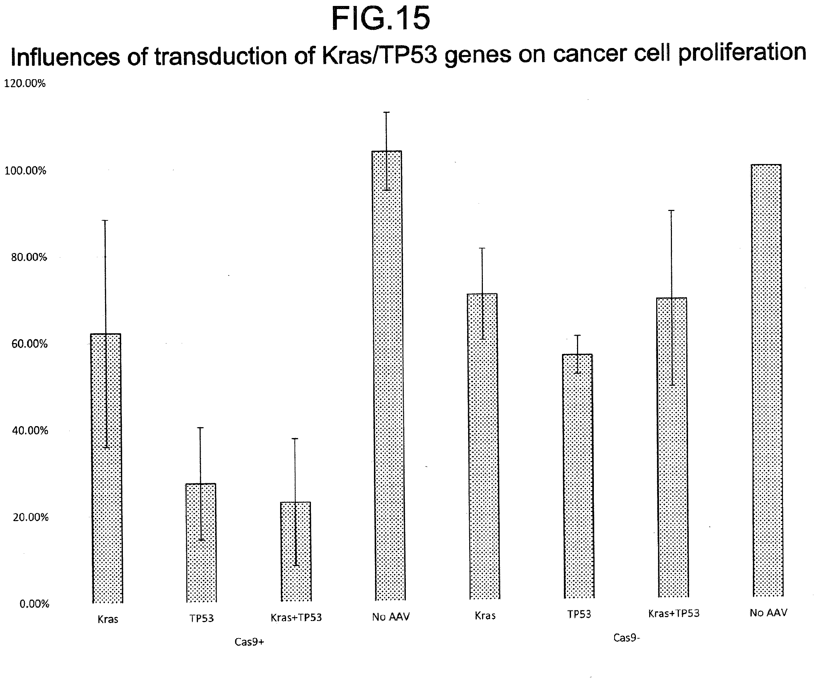

Fig. 15 is illustrative of a totalized cell proliferation of a cancer-derived cell line DLD-1 to which Kras and TP53 were administered.
Fig. 16 is illustrative of a totalized proliferation of normal human colonic epithelia cells to which Kras and TP53 were given.
Fig. 17 is illustrative of measurements by the qPCR method of Kras and TP53 inserted in the genome of cancer cell DLD-1.
Fig. 18 is illustrative of measurements of cell death situations in terms of cancer cell DLD-1 to which Kras and TP53 were given.

### Embodiments for Carrying Out the Invention

Some embodiments of the invention are now explained with reference to the accompanying drawings. The present invention has for its object to provide a viral vector that proliferates and expresses specifically in a cancer cell to have an antitumor action, and a cancer cell proliferation inhibitor comprising the same.

Whereas gene therapy for colorectal cancer heretofore comprises transduction of genes into cancer cells via a viral vector for the purpose of inhibiting the progression of cancer cells via transient expression of genes in the cells, the present invention has for its purpose to use a non-pathogenic viral vector for direct genome edition thereby recovering the function of genomes.

Genome mutations occur through somatic replication errors, medicines, physical damages, etc., and if such mutations are accumulated up to a constant level without repairment, it gives rise to malignant transformation. Numerous knowledges about analysis of the mechanism of carcinogenesis and accumulation of gene mutations regarding colorectal cancer included in numerous types of cancers have been reported so far in the art. These analyses tell that the number of gene mutations found in varying colorectal cancer cells differs depending on cell classes, yet mutations in the major signal transmission path taking part in carcinogenesis are common (Mouradov et al. 2014).

In the present invention, therefore, Kras, TP53 and APC are selected as common mutant genes found in different types of colorectal cancers to recover the functions of Kras and TP53 in the cancer cells for the purpose of inhibiting cancer cell proliferations.

It is here understood that the "gene" or "DNA" used herein does not only refer to double-stranded DNAs but also to DNAs including a single-stranded DNA such as a sense chain or an antisense chain. There is no particular limitation on their length. In other words, the "gene" or "DNA" used herein comprises a double-stranded DNA including a genomic DNA, a single-stranded DNA (positive strand) including cDNA, and a single-stranded DNA having a sequence complementary to the positive strand (complementary strand or reverse strand) as well as synthetic DNAs, if otherwise stated. Further, RNAs are included in the "gene" too. It is also noted that the "gene" or the "DNA" includes, in addition to a code area, other areas such as expression control area, signal area, exon, and intron on condition that the advantages of the invention are maintained just the way they are.

Unless otherwise stated, the "gene" or "DNA" referred to herein includes a "gene" or "DNA" represented by a specific base sequence as well as a "gene" or "DNA" capable of coding protein equivalent to the protein coded thereby in terms of physiological function.

The "vector" refers to a DNA molecule for delivery of nucleic acid (or acids) to a host cell which may include, as an example but not limited to, a virus, a plasmid, a phage, and other DNA molecule which is capable of replicating or being replicated in vitro or a host cell or, alternatively, delivering the desired DNA segment to the desired location in a host cell. The vector includes a nucleic acid that codes the substance to be delivered, facilitating addition of the nucleic acid into the host cell and/or replication of the vector in the host cell; it may selectively contain a viral capsid or other substance (for instance, a reverse transcriptase or other enzyme packaged in the capsid or as part thereof).

Reference will now be made to how to use the viral vector according to the invention.

The viral vector described herein may be used as an anti-cancer cells vector for the purpose of, for instance, simple cancer treatment as well as prevention of post-surgeon recurrence, prevention of transfer and/or other prevention.

The viral vector disclosed herein may be applied to all kinds of cancers, and particularly to solid cancers in the stomach, the large intestine, the lung, the liver, the prostate, the pancreas, the gullet, the bladder, the gallbladder, a bile duct, the breast, the womb, the thyroid, the ovary, and so on.

The viral vector disclosed herein may not only be applied to an affected site as such, but may also introduced in a living body or organism (the cells or organs of interest) by every known method such as injections via a vein, a muscle, the abdominal cavity, the skin and so on, nasal inhalation, oral inhalation, lung inhalation, oral administrations, suppositories, external preparations.

The viral vector of the invention introduced in the cancer cells in the living body is allowed to proliferate in such cancer cells thereby inhibiting proliferation thereof, leading to cancer treatments or prevention of cancer metastasis.

The viral vector disclosed herein may also be prepared for easy handling by processes such as a freezing process, and then formulated as a medical composition possibly after mixing with a pharmaceutically allowable known carrier such as excipients, extenders, binders, and lubricants and well-known additives (such as buffers, isotonic agents, chelating agents, coloring agents, preservatives, perfumes and sweetening agents).

The viral vector disclosed herein may be administrated orally or non-orally depending on oral forms forms such as tablets, capsules, powders, particles, pills, and syrups, and non-oral forms such as injections, external agents, and eye drops. Preference is given to local injection into muscles, the abdominal cavity or the like, and injection into veins.

An adeno-associated viral vector (AAV) capable of forming a single-stranded DNA in cells may be preferably used as the viral vector disclosed herein. The adeno-associated viral vector (AAV) is substantially free of immunogenicity, gives rise to only weak immunoreactions, and is found to be free of pathogenicity. This viral vector is suitable for gene therapy and has already been used for some gene therapy, because the timing of transducing DNA genomes into cells is independent on cell cycles. The adeno-associated viral vector is characterized by being free of pathogenicity, and being enough enhanced in terms of safety.

Further, the present invention is characterized in that a DNA sequence specially designed for use in genomic DNA insertion or integration in such a way to prevent side effects from taking place while invalidating the function of mutant genes and the function of normal genes and having no influences on a transcription-and-translation frame is added to the first and last of TP53 and APC gene full-length sequence for incorporation in AAV.

According to the invention, nuclease capable of cutting off a specific site of a genomic DNA may be combined with a viral vector capable of generating a short-stranded viral vector to insert a gene sequence having a normal function into a cell genome thereby restoring the gene function for the purpose of inhibiting proliferation of cancer cells.

According to the invention, genome editing for Kras, TP53 and APC is used to prepare a safe vector usable for gene therapy capable of restoring some gene functions present in the genome of cancer cells. It is thus expected that the present invention would have a much more enhanced effect on inhibition of proliferation of colorectal cancer cells.

If, according to the present disclosure, a smaller number of genes are introduced in cancer cells having a lot more gene mutations built up therein, cell deaths in the cells are restored to inhibit abnormal proliferation, resulting in efficacies higher as compared with conventional gene therapy. Further, it is expectable that the cancer cell proliferation inhibitor according to the present invention, because of producing less side effects than the therapy used so far, would achieve more efficacious gene therapy by repeated administrations.

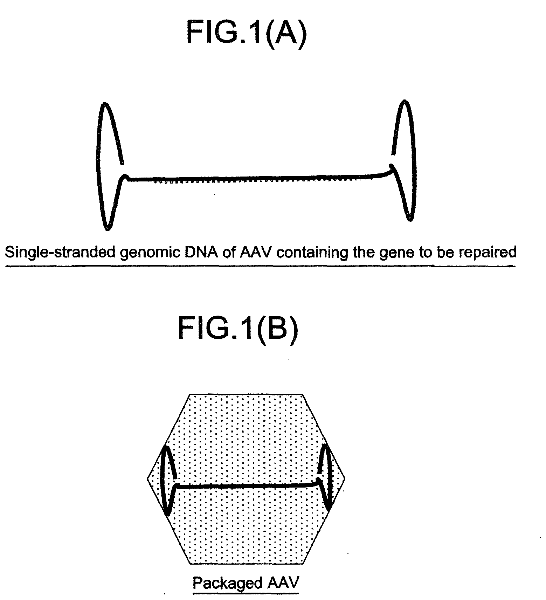

Fig. 1 is illustrative of the adeno-associated viral vector (AAV) according to the present invention; Fig. 1(A) is indicative of a single-stranded genome DNA containing the gene to be repaired, and Fig. 1(B) is indicative of an adeno-associated viral vector (AAV) having said single-stranded genome DNA packaged therein.

The viral vector for gene therapy according to the present invention is integrated or incorporated into the exon site of the target gene on the genome of cancer cells before and after double-stranded DNA fragments having a full-length DNA sequence of the target genes Kras and TP53 so that specific DNA fragments comprising a length of custom-designed 30-35 bases are coupled together to integrate a DNA sequence containing the target genes into the multi-cloning site of the adenovirus associated vector, and further packaged by HEK293 cells in a viral particle comprising a single-stranded DNA protein capsid. Thus, the viral vector of the invention is characterized in that as it is administrated to cancer cells together with nuclease capable of cleaving a specific site of the target gene on the genome, it causes the function of some mutated gene to return to normal without having any impact on the function of normal cells, leading to inhibition of cancer cell proliferation and promoted cell death.

In the invention disclosed herein, an adeno-associated viral vector (AAV) capable of forming a single-stranded DNA in cells is used as the viral vector adapted for gene therapy. The adeno-associated viral vector (AAV) is substantially free of immunogenicity, gives rise to only weak immunoreactions, and is found to be free of pathogenicity. This viral vector is suitable for gene therapy; it has already been used for some gene therapy, because the timing of introducing DNA genomes into cells is independent on cell cycles. This vector is enhanced in itself in terms of safety; recombination experiments using the adeno-associated viral vector may be carried out even at BCL1 grade facilities.

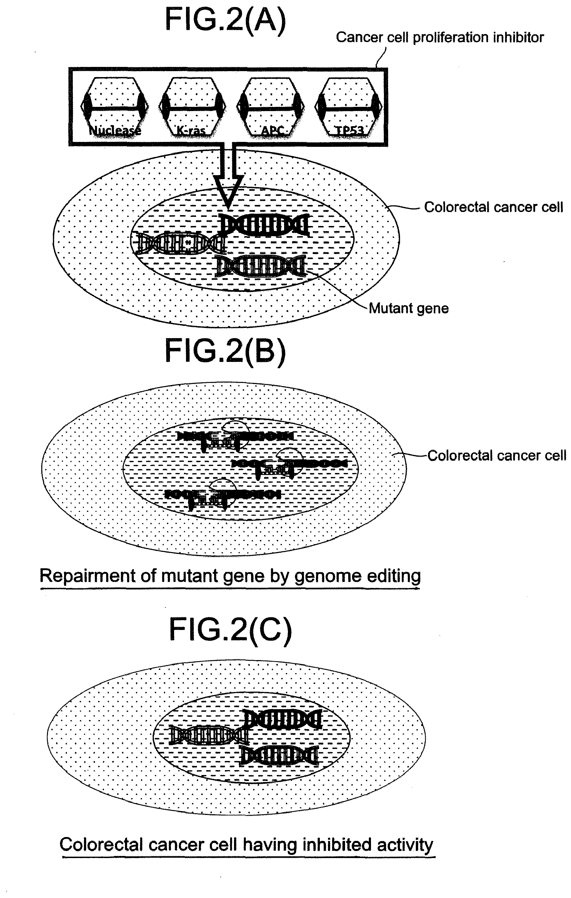

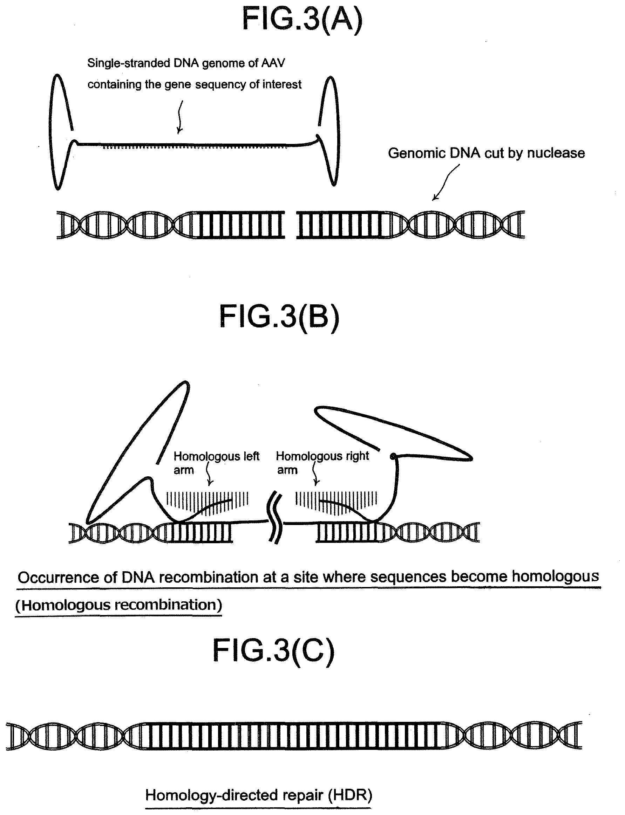

Fig. 2 is schematically illustrative of the mechanism of the cancer cell proliferation inhibitor according to the present invention. The cancer cell proliferation inhibitor according to the present invention may comprise an adeno-associated viral vector (AAV) having a single-stranded genome DNA packaged therein, and a DNA nuclease. Fig. 2(A) is indicative of how the cancer cell proliferation inhibitor is introduced in a colorectal cancer cell having a mutant gene. As the cancer cell proliferation inhibitor is introduced into a colorectal cell, it allows the mutant gene to be restored by genome editing on the basis of the single-stranded genome DNA and DNA nuclease as shown in Fig. 2(B), and inhibits the activity of colorectal cancer cells as shown in Fig. 2(C). Fig. 3 is illustrative of the mechanism of repairing mutant genes.

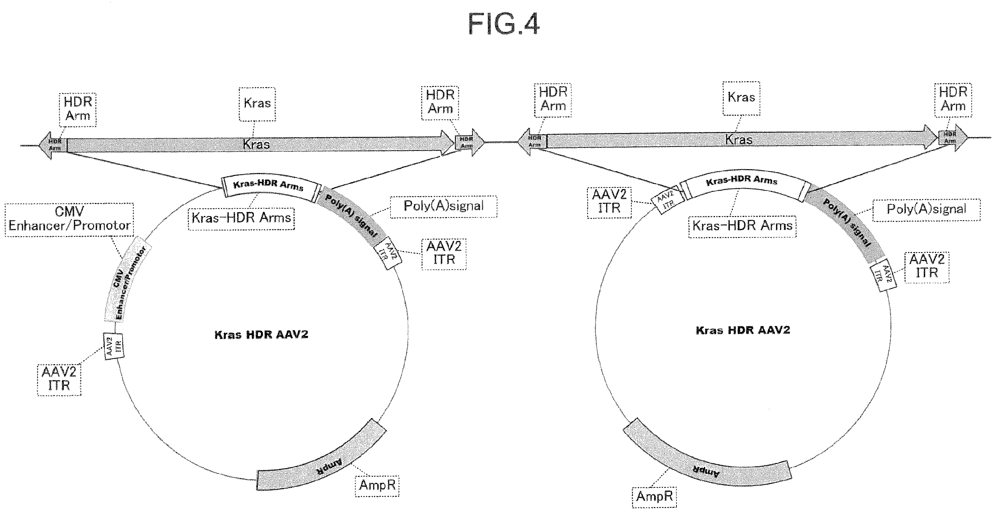

Fig. 4 is indicative of two types of adeno-associated viral vectors (AAV) having Kras integrated therein. A vector having a CMV promotor could synthesize a normal genetic protein without being integrated into the genome of cancer cells; however, a vector having no CMV promotor could fail synthesize any normal generic protein without recourse to integration in the genome of cancer cell. The two types of adeno-associated viral vectors needed to conform to the natures of gene and cancer cells may be administrated to cancer cells.

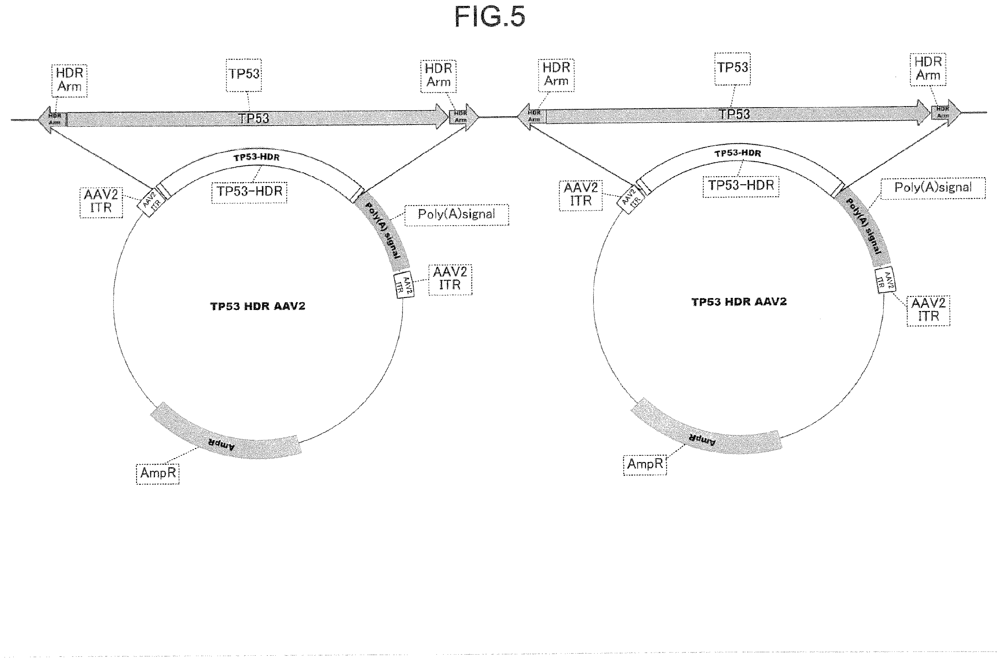

Fig. 5 is illustrative of two types of adeno-associated viral vectors (AAV) having TP 53 integrated therein. As is the case with the adeno-associated viral vector for Kras, there are two types with or without a CMV promotor, which may be used depending on the situations involved.

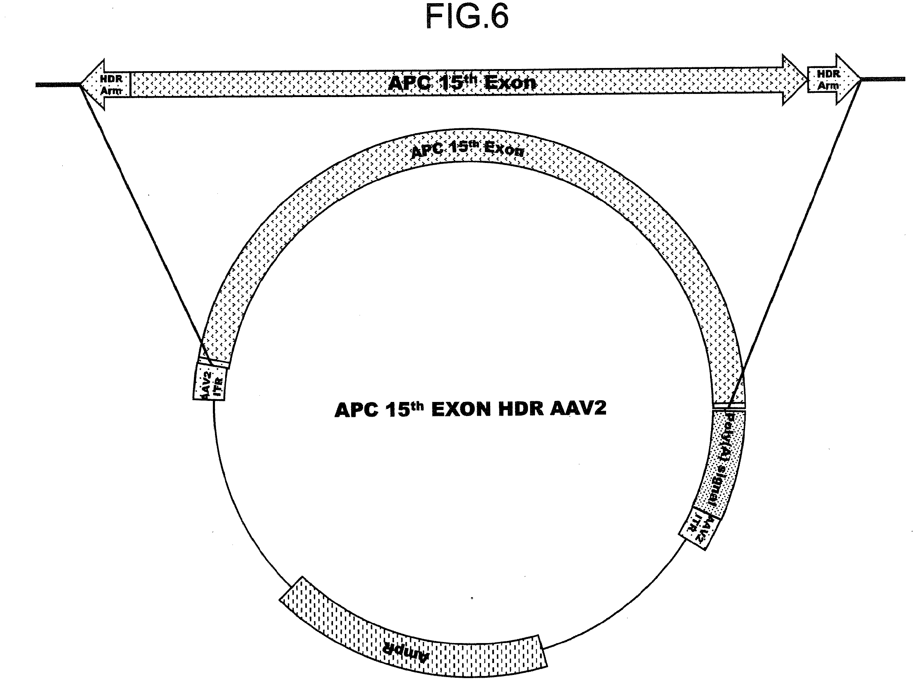

Fig. 6 is illustrative of two types of adeno-associated viral vectors (AAV) having APC integrated therein. The DNA sequence of APC integrated in this adeno-associated viral vector is a DNA sequence up to the last of a region translated from the 15^{th} exson rather than a full-length sequence of APC gene; in other words, only the adeno-associated viral vector having no CMV promotor may be used.

### Vector Construction

As stated above, the adeno-associated viral vector (AAV) is herein selected as the vector used upon administration of gene to a human body while putting emphasis on the safety and efficacy of gene therapy and reductions of side effects. For integration of a predetermined gene sequence for insertion upon gene therapy into an adeno-associated viral vector, the following operations are conducted.

The same sequence as the restriction enzyme recognition sequence found at the multicloning site of the adeno-associated viral vector is added to both ends of the gene sequence to be inserted. (Sall) and (BamHI) are added to the leading and terminal ends of a gene sequence that is to be inserted in the presence of the CMV promotor of the adeno-associated viral vector, and recognition sequences (EcoRV) and (BamHI) are added to the leading and terminal ends of the gene sequence that is to be inserted in the absence of the CMV promotor.

The adeno-associated viral vector and the sequence inserted with restriction enzyme recognition sequence added thereto are treated at 37°C for 2 hours, using the aforesaid restriction enzyme. It is here understood that simultaneously with the restriction enzyme treatment of the adeno-associated viral vector, a dephosphorylation enzyme alkaline phosphatase is used, after which nucleic acid is purified for removal of the protein and enzyme.

DNA Ligation Mix available from Takara-Bio Corporation is added to the vector and insertion sequence that has been purified and treated with the restriction enzyme for nucleic acid coupling reaction under the conditions as described in the DNA Ligation Mix protocol.

After the Ligation reaction, the reaction product is introduced into E. coli, and cultured at 37°C for 16 hours, whereupon the resultant colony is picked up and placed in a 3-ml LB culture medium for 16-hour culture in a shaking environment of 37°C. Thereafter, nucleic acid in the E. coli is purified using the miniprep method to select and store a vector having Kras, TP53 or APC gene sequence contained therein.

### Virus Production

Production of an adeno-associated virus from the adeno-associated viral vector having genes integrated therein is carried out using the AAV Pro System commercialized by Takara-Bio Corporation, and processes of producing, generating and storing adeno-associated viruses from the adeno-associated viral vector are pursuant to the AAV Pro System commercialized by Takara-Bio Corporation.

### Determination of the Genome Site to be Inserted

The present inventors have made study after study with respect to a site into which normal sequences of three genes, say, Kras, TP53 and APC are inserted while the function of a mutant gene in a host cell is disabled by gene editing without doing damage to the function of a normal gene and giving rise to any side effects. Consequently, the inventors have determined the site where a full-length sequence of Kras, TP53 and APC genes is inserted into the genome without having influences on gene transcription and translation frames.

A site where Kras gene was inserted between ccagtactcccggcccccgccattt (Sequence No. 1) and cggactgggagcgagcgcggcgcagg (Sequence No. 2) of Excon 1 of Kras on a host cell genome was determined as the site for insertion.

A site where TP53 gene was inserted between ggacactttgcgttcgg (Sequence No. 3) and gctgggagcgtgctttccac (Sequence No. 4) of Exon 1 of TP53 on a host cell genome was determined as the site for insertion.

Further studies have also been made with regard to a gene insert site used for the purpose of disabling only a part of gene sequences on a genome rather than the full-length of a gene by the length of the gene to be inserted or the features of gene mutations to determine a partial sequence of Kras, TP53 and APC genes having no influence on transcription and translation frames of genes on the genome of a host cell.

Sites: aatgactgaatataaac (Sequence No. 5) and ttgtggtagttggagctggtg (Sequence No. 6) on Exon 2 of Kras on a host cell genome were determined as the Kras gene insert site.

Sites: cacttttcgacatagtg (Sequence No. 7) and tggtggtgccctatgagccgcctg (Sequence No. 8) on Exon 15 of TP53 on a host cell genome were determined as the TP53 gene insert site.

Sites: ccacgggtgtattgtgt (Sequence No. 9) and gacacctataaacttttcca (Sequence No. 10) on Exon 15 of APC on a host cell genome were determined as the APC gene insert site.

### Design of the Fragment to be Inserted

The gene fragment to be inserted on the genome of a host cell must be designed out of way of gene transcription and translation of the host cell; the gene sequence on the genome of the host cell, the gene sequence to be inserted therein, and the sequence needed for genome editing and recognized by an enzyme were designed as follows, as a result of comprehensive considerations thereof.

Set out below are the sequences added to the first and last of the full-length gene sequence for the purpose of inserting a full-length DNA sequence in Exon 1 of each gene on the genome.
Kras: ggcggctcggccagtactcccggcccccgccattt (Sequence No. 11) - [Kras gene full-length sequence + stop codon] - ccgactgggagcgagcgcggcgcaggcact (Sequence No. 12)
TP53: tgctgggctccggggacactttgcgttccg (Sequence No. 13) - [TP53 gene full-length sequence + stop codon] - gctgggagcgtgctttccacgacggtgaca (Sequence No. 14)

Set out below are the sequences added to the first and last of the gene sequence for the purpose of inserting a normal gene sequence in the mutation multiple sites of the respective genes on the genome.
Kras: gcctgctgaaaatgactgaatatatac (Sequence No. 15 - [Kras gene partial sequence + stop codon] - ttgtggtagttggagctggtggcgtaggca (Sequence No. 16)
TP53: tggatgacagaaacacttttcgacatagtg (Sequence No. 17) - [TP53 gene partial sequence + stop codon] - tcgtggtgccctatgagccgcctgag (Sequence No. 18)
APC: tttgacaatatagacaatttaagtcccacg (Sequence No. 19) - [APC gene partial sequence + stop codon] - gcatctcatcgtagtaagcagagacacaag (Sequence No. 20)

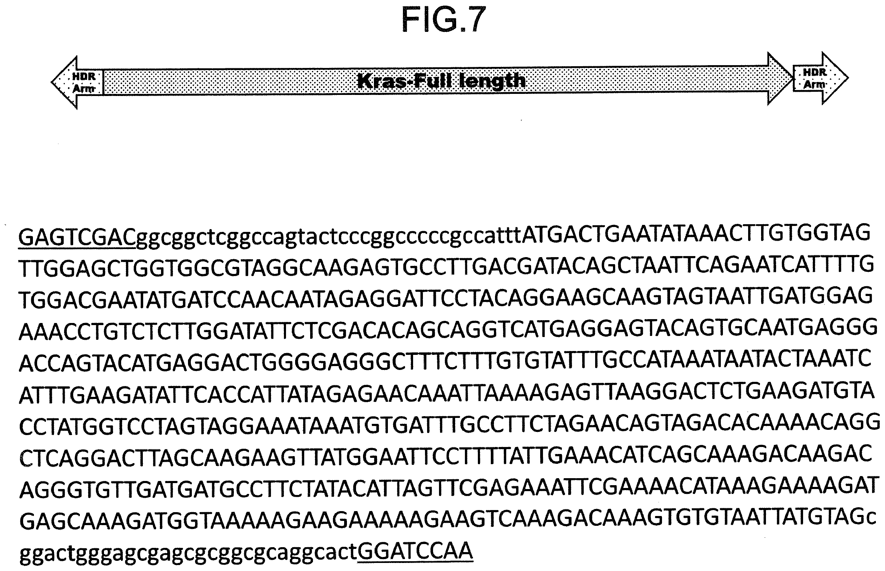

Fig. 7 is illustrative of the full-length sequence of Kras coupled to a special sequence (indicated by lower-case letters) designed for the purpose of integration in the first exon of Kras on the genome of a cancer cell. It is here noted that a sequence of 8 bases (indicated by 8 upper-case letters) forward or rearward of the special sequence designed for integration in genome provides a restriction enzyme site.

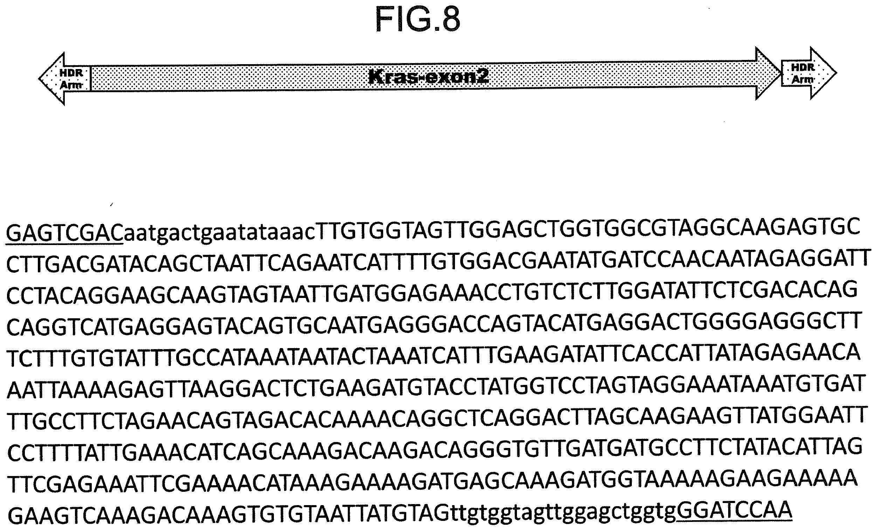

Fig. 8 is illustrative of the full-length sequence of Kras coupled to a special sequence (indicated by lower-case letters) designed for the purpose of integration in the second exon of Kras on the genome of a cancer cell. It is here noted that a sequence of 7 bases (indicated by upper-case letters) forward or rearward of the special sequence designed for integration in genome provides a restriction enzyme site.

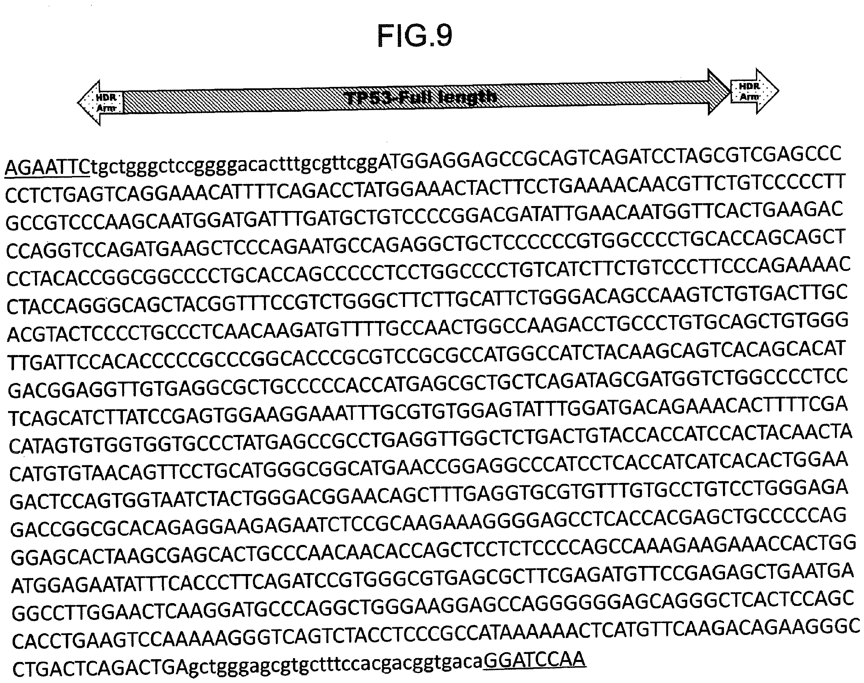

Fig. 9 is illustrative of the full-length sequence of TP53 coupled to a special sequence (indicated by lower-case letters) designed for the purpose of integration in the first exon of TP53 on the genome of a cancer cell. It is here noted that a sequence of 8 bases (indicated by upper-case letters) forward or rearward of the special sequence designed for integration in genome provides a restriction enzyme site for integration of a synthesized DNA fragment in a region called a multicloning site of the adeno-associated viral vector.

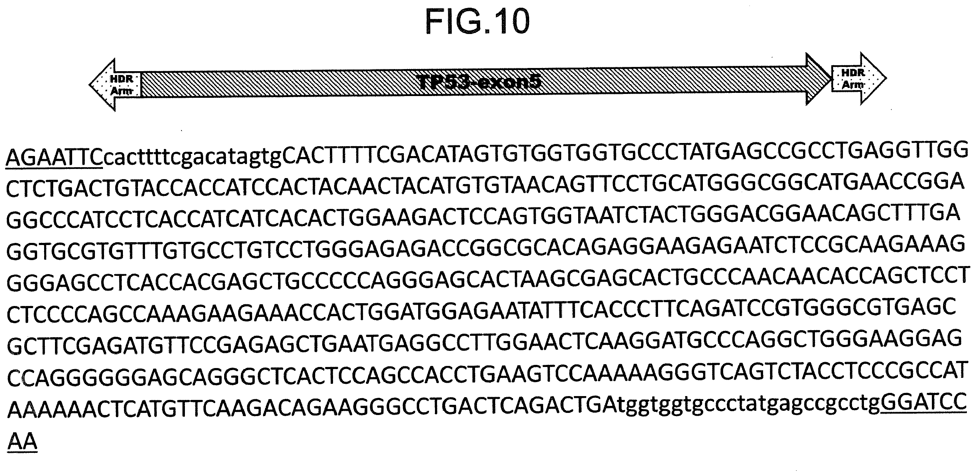

Fig. 10 is illustrative of a partial DNA sequence of TP53 coupled to a special sequence (indicated by lower-case letters) designed for the purpose of integration in the fifth exon of TP53 on the genome of a cancer cell. The fifth to eighth exons of TP53 in the cell provide a region where cancerization-deriving mutations occur frequently; this adeno-associated viral vector is prepared for the purpose of replacing a sequence of the portion corresponding to the fifth exon up to the protein-coding last with a normal sequence thereby recovering the function of muted TP53 in the cancer cell. The adeno-associated viral vector having a full-length sequence TP53 contained therein, and the adeno-associated viral vector containing only a sequence of the fifth exon and later of TP53 may be administrated depending on cancer cell genome states. It is here noted that a sequence of 8 bases (indicated by upper-case letters) forward or rearward of the special sequence (indicated by lower-case letters) designed for integration in genome provides a restriction enzyme site.

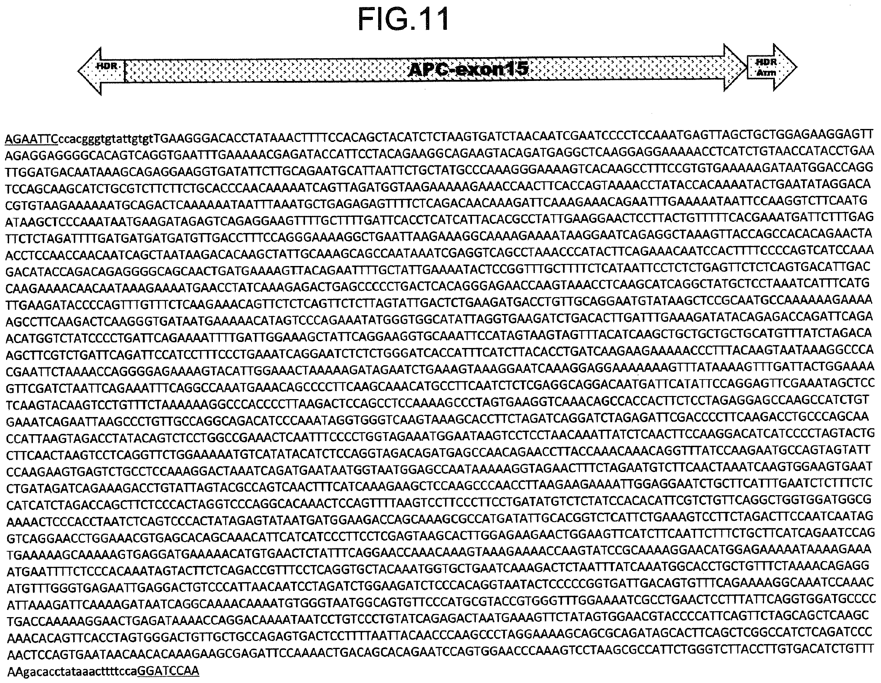

Fig. 11 is illustrative of a partial DNA sequence of APC coupled to a special sequence (indicated by lower-case letters) designed for the purpose of integration in the 15^{th} exon of APC on the genome of a cancer cell. The full-length sequence of APC contains 8000 or more bases; efficiency of recombination to the DNA having a full-length DNA sequence genome is considered considerably low. Containing 3000 bases, the sequence of the 15^{th} exon in the cell genome does not only include substantially a major portion of the APC sequence but also provides an area where cancerization-deriving mutations are frequently found; replacement of the 15^{th} exon and later sequence may lead to recovery of the function of muted APC in the cancer cell. It is here noted that the lower-case letters are indicative of a special sequence designed for integration in the genome, and a sequence of 8 bases (indicated by upper-case letters) forward or rearward of the special sequence provides a restriction enzyme site.

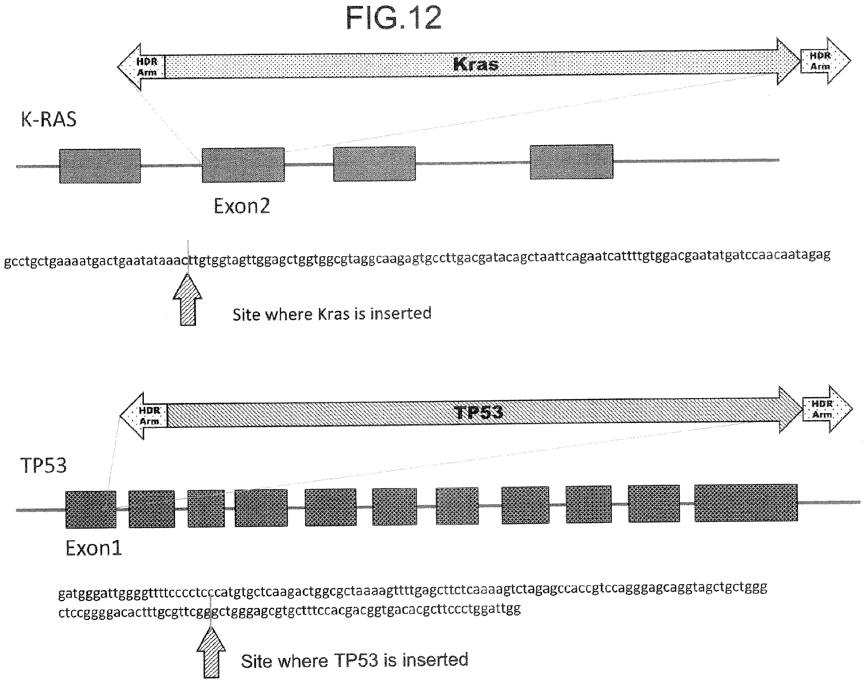

Fig. 12 is schematically indicative of the positions of a genome DNA in which Kras and TP53 are inserted. An adeno-associated viral vector integrated with Kras in such a way as to insert Kras genes in sites: aatgactgaatataaac (Sequence No. 21) and ttgtggtagttggagctggtg (Sequence No. 22) on Exon2 of Kras on a cell genome is applied to a cancer cell simultaneously with nuclease capable of cutting said sites. In the invention disclosed herein, Cas9 and a guide RNA indicative of a cutting site at Cas9 were used. An adeno-associated viral vector integrated with TP53 in such a way as to insert TP53 genes in sites: gacactttgcgttcgg (Sequence No. 23) and gctgggagcgtgctttccac (Sequence No. 24) on Exon1 of TP53 on a host cell genome are given to a cancer cell simultaneously with nuclease capable of cutting said sites.

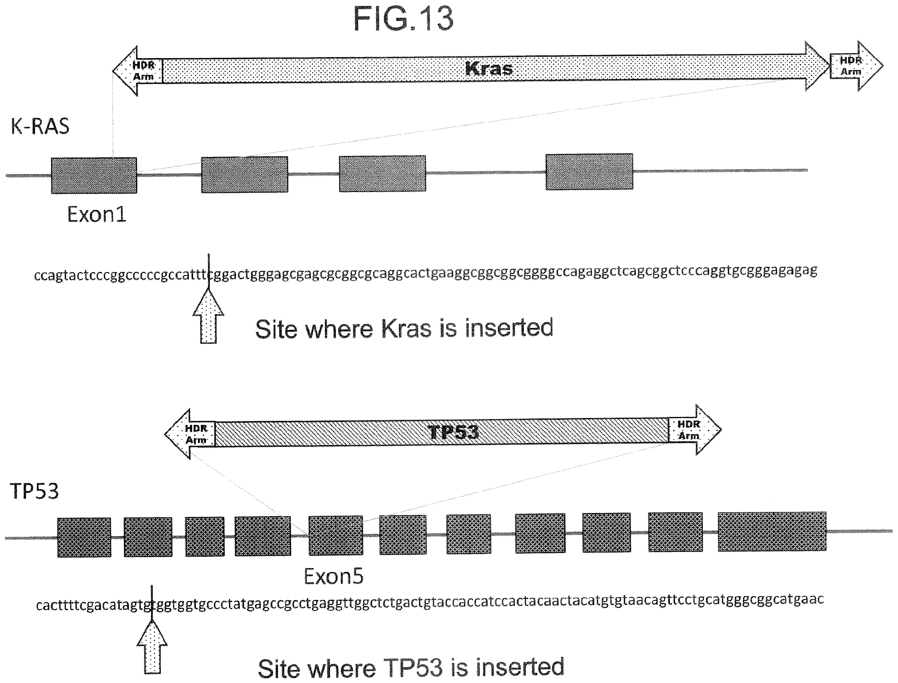

Fig. 13 is schematically indicative of the positions a genome DNA in which Kras and TP53 are inserted. An adeno-associated viral vector integrated with Kras in such a way as to insert Kras genes in sites: aatgactgaatataaac (Sequence No. 25) and ttgtggtagttggagctggtg (Sequence No. 26) on Exon2 of Kras on a cell genome is applied to a cancer cell simultaneously with nuclease capable of cutting said sites. In the invention disclosed herein, Cas 9 and a guide RNA indicative of a cutting site at Cas 9 were used. An adeno-associated viral vector integrated with TP53 in such a way as to insert TP53 genes in sites: cacttttcgacatagtg (Sequence No. 27) and tggtggtgccctatgagccgcctg (Sequence No. 28) on Exon2 of Kras on a cell genome are given to a cancer cell simultaneously with nuclease capable of cutting said sites. In the invention disclosed herein, Cas9 and a guide RNA indicative of a cutting site at Cas9 were used.

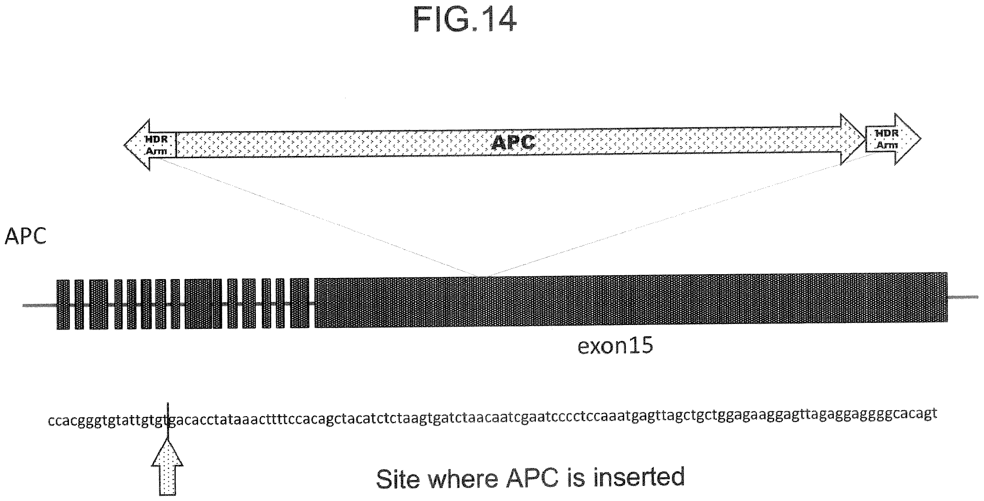

Fig. 14 is schematically indicative of the position of a genome DNA in which APC is inserted. An adeno-associated viral vector integrated with APC in such a way as to insert APC genes in sites: ccacgggtgtattgtgt (Sequence No. 29) and gacacctataaacttttcca (Sequence No. 30) on Exon 15 of APC on a cell genome is applied to a cancer cell simultaneously with nuclease capable of cutting said sites. In the invention disclosed herein, Cas 9 and a guide RNA indicative of a cutting site at Cas 9 were used.

Fif. 15 is indicative of aggregation of cell proliferation of OLD-1 that is a human colon carcinoma cell line to whick Kras and TP53 were administrated. It has been found that as both the DNAs of Cas9 and Kras as well as TP53 genes are transduced into the DLD-1 cells that are human colon carcinoma cell lines, it inhibits proliferation of cells. In particular, it has been observed that the number of cells having TP53 transduced therein is more reduced than a control group. It has also been observed that the cells having Kras transduced therein are reduced in terms of cell proliferation although Kras is less effective than TP53. The cells having both Kras and TP53 given thereto are smaller in number than cells having only TP53 added thereto, meaning that simultaneous administration of both would be more enhanced in terms of inhibition of cell proliferation as compared with single administration. On the other hand, the cells having Cas9 and both CDS and DNA transduced therein have been found to have a more increased effect on inhibition of cell proliferation than cells to which only an AAV vector having CDS and DNA integrated therein, indicating that replacement of genes muted by transduction of normal genes could lead to inhibition of abnormal proliferation of cancer cells. It is here noticeable that there is no significant difference between cells having only Cas9 and guide RNA given thereto and a control group in terms of cell number.

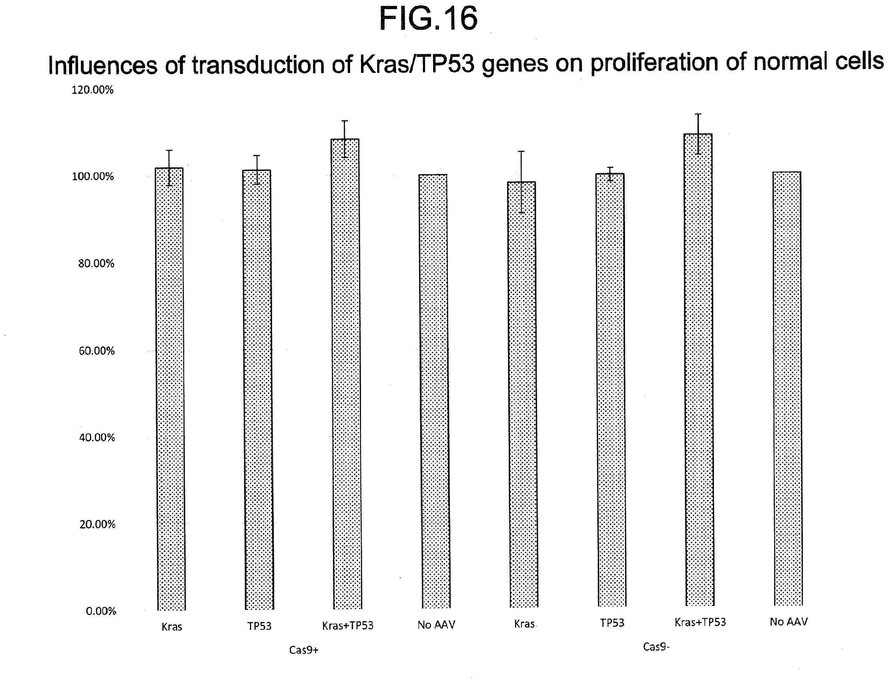

Fig. 16 is indicative of an aggregation of proliferation of normal human intestinal epithelial cells to which Kras and TP53 are given. There is no significant difference in terms of cell proliferation between this group and a control group, meaning that the invention disclosed herein would have no influence on proliferation of normal cells.

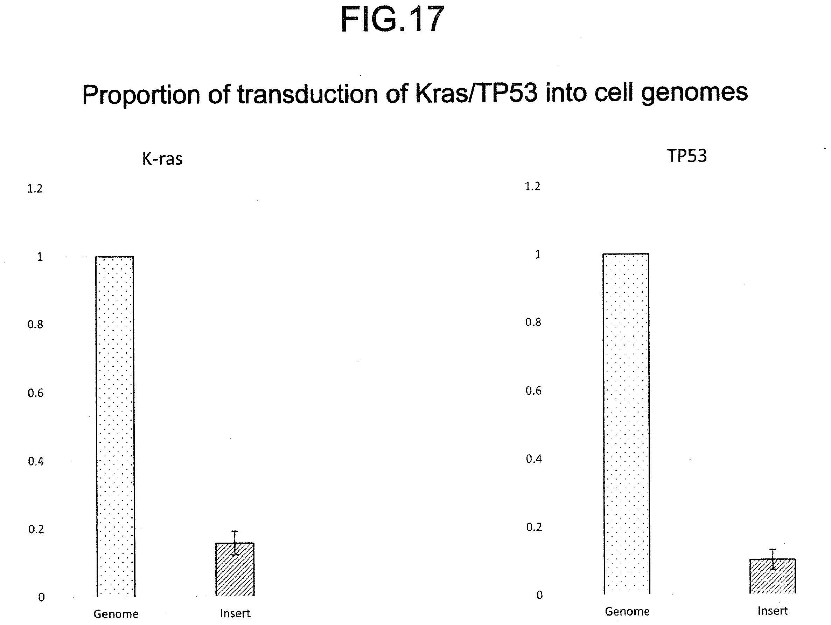

Fig. 17 is illustrative of Kras and TP53 inserted in the genome of cancer cell DLD-1 and measured by the qPCR method. To make sure of the efficiency of allowing the adeno-associated viral vector having nuclease and the target gene integrated therein to cause homologous recombination in the cell, a special sequence permitted to be present only when a gene is integrated in the genome, and a target gene-free portion of the genome sequence was measured by the qPCR method to compare a site possibly expected to have a gene by homologous recombination with a site where no homologous recombination occurs in terms of quantity in the cell. From the results, it has been understood that the quantity of DNA integrated by homologous recombination in the desired site is about 10% on the whole.

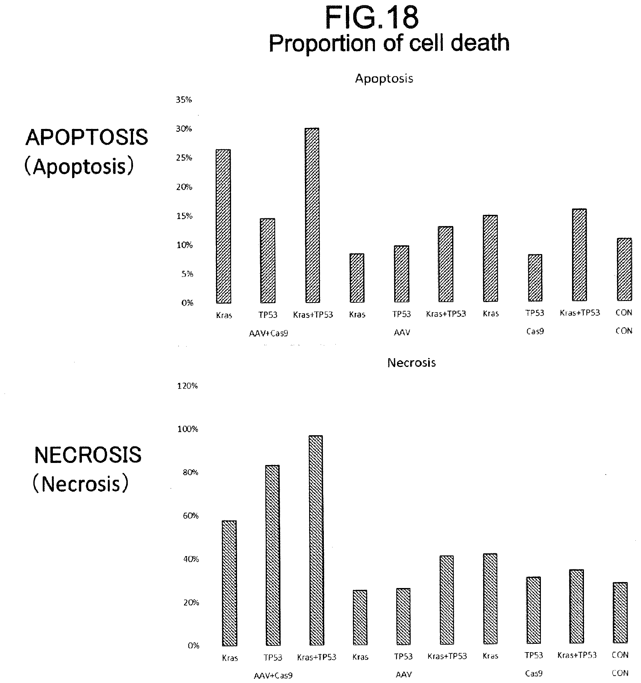

Fig. 18 is indicative of results of measuring cell deaths in cancer cell DLD-1 to which Kras and TP53 were given. To study the cause of cancer cell proliferation inhibition in greater details, the proportion of different cell deaths such as apoptosis and necrosis taking place in cells having Kras and TP53 genes transduced therein were investigated. As a result, it has been found that the proportion of apoptosis and necrosis in the cells having the genes transduced therein is higher as compared with a control group. It has clearly been seen that the proportion of apoptosis in the cells having only Kras transduced therein increases, whereas the proportion of necrosis in the cells having only TP53 transduced therein increases, suggesting that the respective genes have an action on cancer cell proliferation inhibition. Furthermore, it has been clear that the cells having both Kras and TP53 genes transduced therein lead to an increase of both apoptosis and necrosis, indicating that simultaneous transduction of Kras and TP53 in the cancer cells gives rise to an increase in varying cell deaths among the cancer cells, resulting in the synergistic effect on cancer cell proliferation inhibition due to Kras and TP53. In the cells in which only genes are transduced to prevent gene recombination to the cancer cell genome, it has been found that there is no noticeable increase in the proportion of cell deaths, showing that the effect of the present invention on cancer cell proliferation inhibition is higher than a generally available transgenesis method.

### Verification of the Effects of the Invention

To verify reversible possibility while cancer cells are varied in state by the present invention, the adeno-associated viral vector constructed with genes integrated therein was used in combination with a genome editing method capable of cutting a specific site on the genome for insertion of the genes in the cell genome, after which cell states were analyzed to verify whether they could be varied by recovery of gene functions.

Colorectal cancer was selected as the cell for verification from among numerous genetic diseases. Causes of colorectal cancer and accumulations and changes of genetic mutations have long been studied and investigated, and clarification of the cause and mechanism of cancerization is now well going. Colorectal cancer may be generally classified to four patterns depending on types and frequencies of genetic mutations.

In the invention disclosed herein, APC, Kras and TP53 are picked up as common, major cancer genes from four such patterns. Kras and TP53 suitable for transduction from three such genes into AAV vectors were used to construct a DNA repair method, followed by the verification of efficacy of genetic therapy for cancer cells.

Referring here to integration of the full-length CDS of these genes into Exon1 by homologous recombination, it is designed in such a way that CDS having a normal sequence is not transcribed, resulting in translation of an endogenous gene with functional abnormality. To confirm the efficacy of causing CRISPR/Cas9 and AAV vector to bring about homologous recombination in a cell, PCR was applied to cells in which CRISPR/Cas9, guide RNA for setting a target site, and CDS DNA of the gene were all transduced to compare a site expected to have genes transduced by homologous recombination with a site unlikely to be subjected to homologous recombination in terms of quantity in the cell. From the results of qPCR, it has been found that the DNA integrated by homologous recombination into the desired site is about 10% on the whole.

As a result of transduction of both DNAs containing CDS: the protein coding region of CRISPR/Cas9, Kras and TP53 genes into Lovo cells or a cell line derived from human colorectal cancer cells, it has been found that cell proliferation is inhibited. Referring to a cell having TP53 transduced therein in particular, it has been observed that the number of cells is much less than that of a control group.

Even the cells having Kras transduced therein are less than a control group in terms of number although making comparisons with TP53 difficult. The cells having both CDS DNAs: Kras and TP53 applied thereto are less than in number cells having only TP53 applied thereto; this would mean that simultaneous application of both is more than a single application in terms of cell proliferation inhibition effect.

Referring here to the cells having both CRISPR/Cas9 and CDS DNA transduced therein, they are much more enhanced than the cells to which only the AAV vector CDS DNA integrated therewith is applied in terms of cell proliferation inhibition effect, indicating that replacement of muted genes rather than transduction of normal genes is more efficacious for cell proliferation inhibition. It is here noted that there were no significant differences in number between the cells having only CRISPR/Cas9 and guide RNA (gRNA) given thereto and a control cell group.

To observe how the cells behave after transduction of genes, observations were made of cells where homologous recombination took place upon transduction of TP53 comprising GFP (green fluorescent protein) coupled to an AAV vector together with CRISPR/Cas9.

At this time, a CMV promotor on the AAV vector was removed in such a way as to prevent expression of any unrecombined genes. Twenty-four (24) hours after transduction of TP 53, the cells were observed to emit fluorescence out of GFP; however, it has been found that signals of GFP in the cell decrease with the elapse of time.

In particular, the GFP signal number of the cell having TP53 transduced therein disappeared nearly after 72 hours after transduction. In contrast, it has been observed that in the cells having a control transduced therein, there is an increase in the GFP signal number, indicating an influence of gene recombination on cell proliferation. The GFP signal number was much less than the result of PCR; post-24 hours observation with an increasing amount of the viral vector has indicated that no substantial GFP signal is observed in the cell having TP53 transduced therein, and cell proliferation is considerably inhibited as well.

Referring then to the cell where the DNA vector having no influence on cells is transduced in, it has been presumed that there is a GFP signal observed with no inhibition of cell proliferation; TP53 acts on cell proliferation inhibition before accumulated up to an observable quantity, leading to apoptosis. It has been understood that Kras is of poor efficiency upon simultaneous transduction of genes, often failing to observe GFP signals.

To go further into the cause of cancer cell proliferation inhibition, studies were made of various types of cell deaths induced in the cells having genes Kras and TP53 transduced therein such as apoptosis and necrosis. It has consequently been found that the gene-transduced cells are more increased than a control cell group in the proportion of apoptosis and necrosis. It has also been seen that the proportion of apoptosis grows larger in the cell having only Kras transduced therein whereas the proportion of necrosis grows larger in the cell having only TP53 transduced therein, suggesting that the respective genes have an action on cancer cell proliferation inhibition.

Further, it has been seen that both apoptosis and necrosis grow large in the cells having both Kras and TP53 transduced therein, making it clear that simultaneous transduction of Kras and TP53 in cancer cells causes different types of cell deaths in the cancer cells to increase all at once: a principal cause of synergistic effect of cancer cell proliferation inhibition by Kras and TP53. It has also been seen that there is no significant increase in cell deaths in the cells having only the genes transduced therein such that any gene recombination relative to cancer cell genomes does not come along; in other words, the effect of cancer cell proliferation inhibition by the inventive method is higher than a general gene transduction.

From verifications according the invention disclosed herein, it has been evident that the efficiency of DNA homologous recombination relative to cells is good enough to inhibit cell proliferation.

There has already been a report about the synergistic effect capable of enhancing the efficiency of homologous recombination by CRISPR/Cas9 and AAV vector, wherein it is presumed that the presence of a single-stranded DNA near a genomic DNA cut by endonuclease causes a DNA repair mechanism to be activated and start working, allowing homologous recombination to take place with an efficiency higher than usual. It has also been presumed that a similar phenomenon is induced by cutting not only by Cas9 alone but also by DNA endonuclease.

From the results of verification according to the invention disclosed herein, it has been understood that simultaneous administration of CDS DNA of Kras/TP53 and CRISPR/Cas 9 rather than the presence of CDS DNA of Kras/TP 53 as a vector has a greater influence on cell proliferation inhibition. As the vector is also integrated with a CMV promotor, the gene expression efficiency could not dramatically increase. From the results of analysis of cells having CRISPR/Cas9 and Guide RNA given thereto, it is seen that keeping expression low due to mutation of Exon1 by CRISPR/Cas9 would be little likelihood.

Referring here to the CDS DNA of Kras/TP53, it would have an enhanced influence on cell proliferation inhibition if it is integrated by homologous recombination into the genome rather than present as a vector, because integration of the CDS DNA of Kras/TP53 into Exon1 of Kras/TP53 by homologous recombination could hinder the translation of muted genes, ending up with inhibition of cell abnormal proliferation traceable to genetic mutation of Kras/TP53. The results of verification according to the invention disclosed herein have shown that if homologous recombination is used to integrate a normal gene sequence into the transcription start point of a gene such as Exon1, functional aberrations due to gene mutations in cells can be repaired.

Reportedly, Kras and TP53 are important genes taking part in control of cell cycles, cell proliferation, and cell death. As known in the art, Kras participates in control of apoptosis via the signal path of RAR/ERK or P13K/AKT, and TP53 takes part in control of necrosis caused by stress on cells as well. Recovery of the Kras function inhibits the action of abnormally activated P13K/AKT to enhance necrosis, resulting in returning abnormal cancer cell proliferation back to a normal level. This would be presumed to be the mechanism of action leading to the synergistic effect of Kras/TP53 administration.

From the verification according to the disclosure provided therein, it has been understood that it is possible to inhibit cancer cells from proliferating by using a combination of endonuclease and a single-stranded DNA-generating viral vector for recovery of gene functions.

From the results of verification, it has further been seen that the transduction of only a single gene allows the functions of apoptosis and necrosis in cancer cells to be recovered to inhibit the cancer cells from proliferating abnormally. In view of the developmental mechanism of cancer cells, it has been known that there is the need for accumulation of much more genetic mutations until they grow to cancer cells, and established cancer cells have much more mutations stored via an extended passage culture.

However, the fact that the cancer cells having such attributes are significantly inhibited in terms of cell proliferation if the function of at least one gene is restored suggests that unusual immortality and cell proliferation attributes gained by the cancer cells are neither well-built nor irreversible; gene functions participating in them may be restored back to the same level as normal cells.

If some method for taking advantage of the invention disclosed herein to recover gene abnormality in cancer cells thereby reversibly normalizing the cancer cells is available in the art, this could lead to development of an epoch-making therapy in cancer treatments.

According to the viral vector and cancer cell proliferation inhibitor of the present invention, a genome could be directly edited for cancer cells to repair gene functions resulting in inhibition of cancer cell progression.

Unlike cancer treatment methods available so far in the art, the present invention has for its purpose to make use of a non-pathogenic viral vector while keeping its influence on normal cells to a minimum thereby restoring muted gene functions responsible for rendering cancer cell abnormal; it is possible to establish a safer permanent gene therapy.

### Industrial Applicability

The present invention relates to a viral vector and a cancer cell proliferation inhibitor containing the same. It has so far been desired to establish technology capable of editing a genome directly for cancer cells to repair gene functions thereby inhibiting cancer cell progression. The present invention makes it possible to edit a genome directly for cancer cells while minimizing influences on normal cells thereby inhibiting cancer cell progression.

## Claims

1. A viral vector formed in such a way as to transduce a full-length cDNA having a stop codon of Kras into the first exon of Kras of a human cell genome DNA.

2. A viral vector formed in such a way as to transduce a full-length cDNA having a stop codon of TP53 into the first exon of TP53 of a human cell genome DNA.

3. A viral vector formed in such a way as to transduce a full-length cDNA having a stop codon a site of exon just before a transcription start point of a specific gene into a genome DNA.

4. A viral vector formed in such a way as to transduce a cDNA having a stop codon, corresponding to the second to fourth exon of Kras into the second exon of Kras of a genome DNA.

5. A viral vector formed in such a way as to transduce a cDNA from the fifth exon to the eighth exon of TP53 into the second exon of TP53 of a genome DNA.

6. A viral vector formed such that a cDNA having a stop codon up to the last of a region translated from an MCR site of APC on which a gene mutation is focused is transduced from the vicinity of a start position of the 15^{th} exon of APC of a genome DNA to the last of a protein coding region of a gene.

7. A cancer cell proliferation inhibitor **characterized by** comprising a viral vector according to any one or more of claims 1 to 6, and a DNA nuclease.
